# EUROPÄISCHE PATENTANMELDUNG

(11) **EP 4 159 194 A1**
(43) Veröffentlichungstag der Anmeldung: **05.04.2023**
(21) Anmeldenummer: 22196323.4
(22) Anmeldetag: 19.09.2022
(51) Int. Cl.: A61K 9/00, A61K 47/14, A61K 9/107

(54) **ÄUSSERLICH ANWENDBARE ZUSAMMENSETZUNG VON IBUPROFEN MIT BENZYLNICOTINAT ODER CAPSAICIN**

(30) Priorität: 04.10.2021 DE 102021125624
(71) Anmelder: Dr. Theiss Naturwaren GmbH, 66424 Homburg (DE)
(72) Erfinder: Nardi, Giuseppe, 66424 Homburg (DE)
(74) Vertreter: Zeitler Volpert Kandlbinder Patentanwälte Partnerschaft mbB

(57) **Zusammenfassung**

Die Erfindung betrifft eine äußerlich anwendbare Zusammensetzung mit einer wirksamen Menge Ibuprofen sowie folgenden weiteren Inhaltsstoffen: mittelkettige Triglyceride, Glycerolmonostearat, Poly(oxyethylen)-30-stearat, Poly(oxyethylen)-100-stearat, Wasser, Methyl-4-hydroxybenzoat-Natrium, 1,2-Propandiol, XanthanGummi, wobei die Zusammensetzung ferner Benzylnicotinat oder Capsaicin enthält. Ferner betrifft die Erfindung ein Verfahren zum Herstellen einer solchen Zusammensetzung vorzugsweise in Form einer Creme.

## Beschreibung

Die Erfindung betrifft eine äußerlich anwendbare Zusammensetzung mit einer wirksamen Menge Ibuprofen.

Ibuprofen ist ein häufig verwendetes und häufig verschriebenes nichtsteroidales Antirheumatikum (NSAID) mit einem bekannten Wirksamkeits- und Sicherheitsprofil. Arzneimittel zur topischen Anwendung, die Ibuprofen enthalten, gehören zur pharmakotherapeutischen Gruppe der nichtsteroidalen Antirheumatika, Propionsäurederivate (ATC-Code M02AA13).

Eine Ibuprofen 5% Creme ist beispielsweise aus der EP 0 087 062 A2 bekannt und findet als topische Formulierung z.B. Anwendung für die folgenden OTC-Indikationen, wobei sogenannte OTC(over the counter)-Arzneimittel frei verkäufliche Arzneimittel bezeichnen, die beispielsweise in Apotheken oder anderen Verkaufsstellen ohne ärztliche Verschreibung, d.h. ohne Rezept, verkauft werden können:
- OA (Osteoarthritis), insbesondere der Knie-, Hand- und Sprunggelenke;
- Schwellungen oder Entzündungen der an die Gelenke angrenzenden Weichteile;
- Insertionstendinitis, einschließlich Epicondylitis humeri radialis (Tennisellenbogen), Epicondylitis humeri ulnaris (Golfer-Ellenbogen);
- Schultersteife, Schmerzen im unteren Rücken, Hexenschuss;
- Sport- und unfallbedingte Verletzungen wie Prellungen, Verstauchungen und Zerrungen
- symptomatische lokale Behandlung von leichten bis mäßiges Schmerzen.

Aus der DE 296 80 194 U1 ist ein Gel bekannt, dass als Wirkstoffe Ibuprofen und Benzylnicotinat enthalten kann. Ferner ist in der EP 2 061 432 B1 eine MikroEmulsion mit Ibuprofen und Capsaicin beschrieben.

Der Erfindung liegt die Aufgabe zugrunde, eine äußerlich anwendbare Zusammensetzung der eingangs erwähnten Art zu schaffen, die insbesondere als wirksame Creme besonders stabil und haltbar vorliegen kann: Ferner liegt der Erfindung die Aufgabe zugrunde, ein Verfahren zum Herstellen einer vorgenannten Zusammensetzung anzugeben.

Diese Aufgaben werden erfindungsgemäß einerseits durch eine Zusammensetzung mit den Merkmalen des Patentanspruchs 1 und andererseits durch ein Verfahren mit den Merkmalen des Patentanspruchs 6 gelöst. Vorteilhafte Weiterbildungen sind jeweils in den abhängigen Ansprüchen angegeben.

Die äußerlich anwendbare Zusammensetzung enthält eine wirksame Menge Ibuprofen sowie folgende weitere Inhaltsstoffe:
mittelkettige Triglyceride,
Glycerolmonostearat,
Po ly( oxyethylen)-30-stea rat,
Poly(oxyethylen)-1 00-stearat,
Wasser,
Methyl-4-hydroxybenzoat, Natrium,
1,2-Propandiol,
Xanthan-Gummi,
wobei die Zusammensetzung ferner Benzylnicotinat oder Capsaicin enthält. Im Gegensatz zu der vorerwähnten EP 0 087 062 A2 enthält die erfindungsgemäße Zusammensetzung zum einen keinen Polyoxyethylen-Fettsäureester, zum anderen liegt der Inhaltsstoff Polyoxyethylenstearat erfindungsgemäß in zwei Komponenten, nämlich Poly(oxyethylen)-30-stearat und Poly(oxyethylen)-100-stearat, vor. Dadurch konnte der Wirkstoff Ibuprofen besonders gut gelöst und letztlich konnte die Zusammensetzung so aufbereitet werden, dass diese besonders stabil und haltbar auch in Form einer Creme vorliegen kann. Der Einsatz von letztlich drei Emulgatoren, nämlich Glycerolmonostearat, Poly(oxyethylen)-30-stearat, Poly(oxyethylen)-100-stearat, ermöglicht das Vorsehen einer zusätzlichen Homogenisierstufe und damit eine ausreichende Stabilität der Zusammensetzung auch als Creme. Benzylnicotinat hat durchblutungsfördernde und wärmende Eigenschaften. Zu den Anwendungsgebieten der Benzylnicotinat enthaltenden Zusammensetzungen gehören: Rheumatische Beschwerden, Weichteilrheuma, schmerzhafte, entzündliche, degenerative Erkrankungen des Bewegungs- und Stützapparates, der Muskeln, Sehnen, Bänder, Gelenke und der Wirbelsäule. Dadurch, dass Benzylnicotinat Wärme spendet und Poren öffnen bzw. weitstellen kann, gelangt der Wirkstoff Ibuprofen schneller und unmittelbarer an schmerzempfindliche Stellen. Die wärmenden Eigenschaften wirken entspannend und tragen mit dazu bei, die Schmerzempfindlichkeit zu reduzieren. Letztlich kann der Wirkstoff dadurch leichter und schneller aufgenommen werden. Ähnliche Eigenschaften hat auch der Wirkstoff Capsaicin. Dieser ermöglicht einen effektiven Transport von Wirkstoffen, vor allem von entzündungshemmenden und juckreizsowie schmerzstillenden Wirkstoffen.

Gemäß einer bevorzugten Weiterbildung der Erfindung liegen die Inhaltsstoffe wie folgt vor:
2 bis 12 Gew.-%, vorzugsweise 3 bis 8 Gew.-%, besonders bevorzugt 5,0 Gew.-% Ibuprofen,
30 bis 50 Gew.-%, vorzugsweise 35 bis 45 Gew.-%, besonders bevorzugt 41,69 Gew.-% mittelkettige Triglyceride,
4 bis 10 Gew.-%, vorzugsweise 5 bis 8 Gew.-%, besonders bevorzugt 7 Gew.-% Glycerolmonostearat,
1 bis 5 Gew.-%, vorzugsweise 2 bis 3 Gew.-%, besonders bevorzugt 2,4 Gew.-% Poly(oxyethylen)-30-stearat,
2 bis 8 Gew.-%, vorzugsweise 3 bis 5 Gew.-%, besonders bevorzugt 4,2 Gew.-% Poly(oxyethylen)-1 00-stearat,
Rest Wasser (gereinigt),
0,05 bis 0,4 Gew.-%, vorzugsweise 0,1 bis 0,2 Gew.-%, besonders bevorzugt 0,15 Gew.-% Methyl-4-hydroxybenzoat, Natrium,
2 bis 10 Gew.-%, vorzugsweise 3 bis 6 Gew.-%, besonders bevorzugt 5,0 Gew.-% 1,2-Propandiol,
0,1 bis 1 Gew.-%, vorzugsweise 0,2 bis 0,8 Gew.-%, besonders bevorzugt 0,54 Gew.-% Xanthan-Gummi,
0,1 bis 2,5 Gew.-%, vorzugsweise 0,3 bis 1,5 Gew.-%, besonders bevorzugt 0,5 bis 1,0 Gew.-% Benzylnicotinat oder Capsaicin. Eine solche Zusammensetzung ist zum einen in der Lage, den Wirkstoff schnell und gezielt am Wirkungsort einzusetzen, zum anderen ist der Wirkstoff stabil und haltbar in der Zusammensetzung eingebunden, wodurch die Gefahr einer Auskristallisierung verringert ist. Dadurch ist eine gute Resorption des Wirkstoffs gewährleistet.

Gemäß einer anderen Weiterbildung enthält die Zusammensetzung den weiteren Inhaltsstoff Parfüm, vorzugsweise Lavendelöl und Bitterorangenblütenöl, wobei die Zusammensetzung vorzugsweise 0,05 bis 0,18 Gew.-%, bevorzugt 0,08 bis 0,15 Gew.-%, besonders bevorzugt 0,11 Gew.-% Lavendelöl und 0,01 bis 0,12 Gew.-%, bevorzugt 0,04 bis 0,08 Gew.-%, besonders bevorzugt 0,06 Gew.-% Bitterorangenblütenöl enthält. Dadurch erhält die Zusammensetzung ihren spezifischen Duft.

Gemäß einer besonders bevorzugten Weiterbildung liegt die Zusammensetzung in Form einer Creme vor. Diese ermöglicht, den Wirkstoff gezielt aufzutragen und auf dem Zielgebiet in der Nähe des Wirkungsorts zu verreiben bzw. in dieses einzumassieren. Insofern kann der Wirkstoff bei topischer Anwendung das Zielorgan unmittelbarer erreichen. Es ist dadurch möglich, die bei oraler und rektaler Anwendung auftretenden Nachteile zu vermindern. Wie zuvor erwähnt ermöglicht der Einsatz von drei Emulgatoren, nämlich Glycerolmonostearat, Poly(oxyethylen)-30-stearat, Poly(oxyethylen)-100-stearat, die Stabilität der Creme durch eine zusätzliche Homogenisierstufe sicherzustellen. Dadurch kann eine Aufspaltung der Creme in unterschiedliche Phasen weitgehend verhindert werden.

Das erfindungsgemäße Verfahren zum Herstellen einer äußerlich anwendbaren Zusammensetzung der zuvor beschriebenen Art enthält die folgenden Schritte:
(a) Bereitstellen von 30 bis 50 Gew.-% mittelkettige Triglyceride;
(b) Zugeben von 4 bis 10 Gew.-% Glycerolmonostearat, 1 bis 5 Gew.-% Poly(oxyethylen)-30-stearat, 2 bis 8 Gew.-% Poly(oxyethylen)-100-stearat und 2 bis 12 Gew.-% Ibuprofen unter Rühren zu dem Stoff gemäß (a),
nachfolgend Erwärmen des Gemisches unter Rühren auf 60 bis 75 °C;
(c) Lösen von 0,05 bis 2 Gew.-% Methyl-4-hydroxybenzoat, Natrium und 1 bis 5 Gew.-% 1,2-Propandiol in 10 bis 50 Gew.-% gereinigtem Wasser bei 60 bis 75 °C;
(d) Zugeben der wässrigen Lösung gemäß (c) zu der Lösung gemäß (b) unter Rühren zwecks Erhalt einer Emulsion;
(e) Suspendieren von 0,1 bis 1 Gew.-% Xanthan-Gummi, 1 bis 5 Gew.-% 1,2-Propandiol und 0,1 bis 2,5 Gew.-% Benzylnicotinat oder Capsaicin;
(f) Zugeben der Suspension gemäß (e) zu der Emulsion gemäß (d) unter Rühren;
(g) Homogenisieren der Emulsion gemäß (f), Abkühlen der Emulsion unter Rühren. Der Einsatz letztlich von drei Emulgatoren und der Einsatz des Stoffes 1,2-Propandiol jeweils zur Hälfte, d.h. zu 50 %, beim Herstellen der wässrigen Lösung gemäß (c) und der Suspension gemäß (e) ermöglicht letztlich das Herstellen einer stabil und dauerhaft vorliegenden Zusammensetzung, insbesondere auch als Creme.

Gemäß einer bevorzugten Weiterbildung werden im Schritt (c) folgende Unterschritte durchgeführt:
(c1) Lösen von 0,05 bis 2 Gew.-% Methyl-4-hydroxybenzoat, Natrium in 1 bis 5 Gew.-% Wasser;
(c2) Erwärmen von 9 bis 45 Gewichtsprozent Wasser auf 60 bis 75 °C;
(c3) Zugeben von 1 bis 5 Gew.-% 1,2-Propandiol in das Wasser gemäß (c2);
(c4) Mischen der Lösungen gemäß (c1) und (c3).

Dadurch entsteht eine besonders homogene Lösung, die weniger leicht zu Ausfällungen neigt.

Gemäß einer besonders bevorzugten Weiterbildung der Erfindung erfolgen im Schritt (d) ein erstes Homogenisieren über einen Zeitraum von 18 bis 23 Min., anschließend ein Abkühlen der Emulsion auf 50 °C, nachfolgend ein zweites Homogenisieren über einen Zeitraum von 7,5 Min. und anschließend ein Abkühlen der Emulsion auf 42 °C. Wie zuvor bereits angedeutet tragen diese beiden Homogenisierstufen dazu bei, dass die Zusammensetzung, insbesondere in Form einer Creme, stabiler und haltbarer ist. Ausfällungen einzelner Substanzen treten dadurch in aller Regel seltener auf oder können nahezu vollständig vermieden werden.

Gemäß einer anderen Weiterbildung erfolgen im Schritt (g) ein erstes Homogenisieren über einen Zeitraum von 1 Min. und ein zweites Homogenisieren über einen Zeitraum von 5 Min. und das Abkühlen auf 34 °C. Dadurch kann eine wirksame, stabil vorliegende und haltbare Zusammensetzung insbesondere in Form einer Creme erhalten werden.

Vorteilhafterweise wird das Verfahren unter Vakuum, vorzugsweise unter einem Vakuum von 0,4 bis 0,7 bar, durchgeführt. Diese Weiterbildung lässt die einzelnen Verfahrensschritte auf der einen Seite gleichmäßig und schnell ablaufen, denn der oder die Inhaltsstoffe können apparatetechnisch vereinfacht einfach in den jeweiligen Behälter eingesaugt werden. Auf der anderen Seite kann dadurch der Einsatz von Förderpumpen eingeschränkt werden oder sogar vollständig entfallen. Dadurch wird der Verfahrensablauf nicht unerheblich vereinfacht, was sich vorteilhaft auf die Herstellungskosten der erfindungsgemäßen Zusammensetzung, insbesondere in Form einer Creme, auswirken kann.

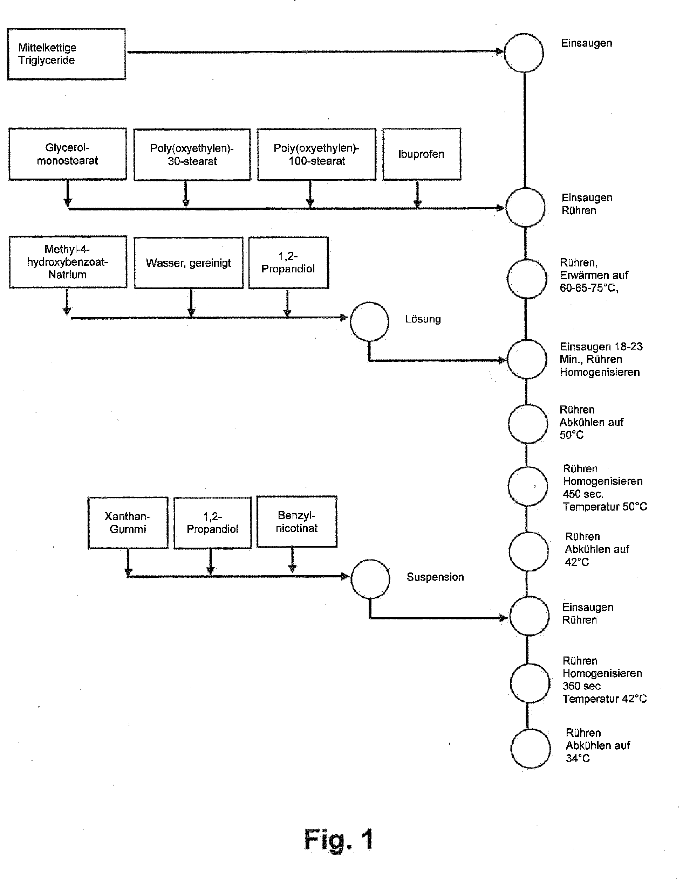

Ausführungsbeispiele des Erfindungsgegenstandes werden nachfolgend auch anhand der Zeichnung näher erläutert, wobei alle beschriebenen und/oder bildlich dargestellten Merkmale für sich oder in beliebiger Kombination den Gegenstand der vorliegenden Erfindung unabhängig von ihrer Zusammenfassung in den Ansprüchen oder deren Rückbeziehung bilden. Es zeigt die einzige Figur
ein Fließbild eines Verfahrens zum Herstellen einer äußerlich anwendbaren Zusammensetzung.

Eine äußerlich anwendbare Zusammensetzung mit einer wirksamen Menge Ibuprofen enthält allgemein wenigstens ein Lösungsmittel, wenigstens einen Emulgator, wenigstens zwei Wirkstoffe, wenigstens ein Konservierungsmittel und wenigstens ein Verdickungsmittel/Stabilisator. Zusätzlich kann die Zusammensetzung ferner wenigstens ein Parfüm, vorzugsweise wenigstens ein Parfümöl, enthalten.

Genauer enthält die äußerlich anwendbare Zusammensetzung mit einer wirksamen Menge Ibuprofen folgende weitere Inhaltsstoffe:
mittelkettige Triglyceride als Lösungsmittel,
Glycerolmonostearat als Emulgator,
Poly(oxyethylen)-30-stearat als Emulgator,
Poly(oxyethylen)-100-stearat als Emulgator,
Wasser als Lösungsmittel,
Methyl-4-hydroxybenzoat, Natrium als Konservierungsmittel,
1,2-Propandiol als Lösungsmittel,
Xanthan-Gummi als Verdickungsmittel/Stabilisator,
wobei die Zusammensetzung ferner Benzylnicotinat oder Capsaicin enthält. Als Wirkstoffe enthält die Zusammensetzung neben Ibuprofen auch die Substanz Benzylnicotinat oder Capsaicin.

Die Eindeutigkeit für pharmazeutische Ausgangsstoffe sind mit der CAS-Nr. (Chemical Abstracts Service) sichergestellt, einem internationalen Bezeichnungsstandard für chemische/pharmazeutische Stoffe. Die Qualität dieser Materialien erfolgt unter Bezug auf Arzneibücher von z.B Pharm Europa, BP (Britisches Arzneibuch), USP (Amerikanisches Arzneibuch) oder Pharm Helvetia.

Nachfolgend sind die vorgenannten Inhaltsstoffe mit den jeweiligen CAS-Nummern angegeben:
Ibuprofen (CAS-Nr. 15687-27-1),
mittelkettige Triglyceride (CAS-Nr. 73398-61-5),
Glycerolmonostearat (CAS-Nr. 85251-77-0, Handelsname Tegin 4100), Poly(oxyethylen)-30-stearat oder Macrogolstearat 1500 (CAS-Nr. 9004-99-3, Handelsname Tego Acid S30),
Poly(oxyethylen)-100-stearat oder Macrogolstearat 5000 (CAS-Nr. 9004-99-3, Handelsname Tego Acid S100),
Methyl-4-hydroxybenzoat, Natrium (CAS-Nr. 5026-62-0),
1,2-Propandiol (CAS-Nr. 57-55-6),
Xanthan-Gummi (CAS-Nr. 11138-66-2),
Benzylnicotinat (CAS-Nr. 94-44-0).

Nähere Informationen betreffend den Wirkstoff Benzylnicotinat werden wie folgt angegeben:
Benzylnicotinat Art.-Nr. 00620890 RNAM, Proben-Nr. 2020-P-92402-1, Ch.B. 158220, F-Ch 19000599, Lieferant Caelo.

Der Wirkstoff Capsaicin ist in einem für die Zusammensetzung eingesetzten Cayennepfeffer-Dickextrakt enthalten und wird wie folgt aufgeführt: Cayennepfeffer-Dickextrakt (4-7:1); Auszugsmittel: Ethanol 80% (V/V) 6,627-18,292 mg pro 1 g Creme = Capsaicin (0,53 mg pro 1 g Creme).

Der Anteil des Wirkstoffs Capsaicin in der erfindungsgemäßen Zusammensetzung beträgt vorzugsweise 0,2 bis 1,2 Gew.-%.

Gemäß einer bevorzugten Ausführungsform der Erfindung liegen die Inhaltsstoffe in der Zusammensetzung wie folgt vor:
2 bis 12 Gew.-%, vorzugsweise 3 bis 8 Gew.-%, besonders bevorzugt 5,0 Gew.-% Ibuprofen,
30 bis 50 Gew.-%, vorzugsweise 35 bis 45 Gew.-%, besonders bevorzugt 41,69 Gew.-% mittelkettige Triglyceride,
4 bis 10 Gew.-%, vorzugsweise 5 bis 8 Gew.-%, besonders bevorzugt 7 Gew.-% Glycerolmonostearat,
1 bis 5 Gew.-%, vorzugsweise 2 bis 3 Gew.-%, besonders bevorzugt 2,4 Gew.-% Poly(oxyethylen)-30-stearat,
2 bis 8 Gew.-%, vorzugsweise 3 bis 5 Gew.-%, besonders bevorzugt 4,2 Gew.-% Poly(oxyethylen)-100-stearat,
10 bis 50 Gew.-%, vorzugsweise 20 bis 40 Gew.-%, besonders bevorzugt 32,85 bis 33,35 Gew.-% Wasser (gereinigt),
0,05 bis 0,4 Gew.-%, vorzugsweise 0,1 bis 0,2 Gew.-%, besonders bevorzugt 0,15 Gew.-% Methyl-4-hydroxybenzoat, Natrium,
2 bis 10 Gew.-%, vorzugsweise 3 bis 6 Gew.-%, besonders bevorzugt 5,0 Gew.-% 1,2-Propandiol,
0,1 bis 1 Gew.-%, vorzugsweise 0,2 bis 0,8 Gew.-%, besonders bevorzugt 0,54 Gew.-% Xanthan-Gummi,
0,05 bis 2,5 Gew.-%, vorzugsweise 0,3 bis 1,5 Gew.-%, besonders bevorzugt 0,5 bis 1,0 Gew.-% Benzylnicotinat oder Capsaicin.

Gemäß einer besonders bevorzugten Ausführungsform enthält die Zusammensetzung den weiteren Inhaltsstoff Parfüm, vorzugsweise Lavendelöl und Bitterorangenblütenöl. Die Zusammensetzung enthält vorzugsweise 0,05 bis 0,18 Gew.-%, bevorzugt 0,08 bis 0,15 Gew.-%, besonders bevorzugt 0,11 Gew.-% Lavendelöl und 0,01 bis 0,12 Gew.-%, bevorzugt 0,04 bis 0,08 Gew.-%, besonders bevorzugt 0,06 Gew.-% Bitterorangenblütenöl.

Die äußerlich anwendbare Zusammensetzung liegt besonders bevorzugt in Form einer Creme vor.

Das erfindungsgemäße Verfahren zum Herstellen einer äußerlich anwendbaren Zusammensetzung insbesondere in Form einer Creme enthält die folgenden Schritte:
(a) Bereitstellen von 30 bis 50 Gew.-% mittelkettige Triglyceride;
(b) Zugeben von 4 bis 10 Gew.-% Glycerolmonostearat, 1 bis 5 Gew.-% Poly(oxyethylen)-30-stearat, 2 bis 8 Gew.-% Poly(oxyethylen)-100-stearat und 2 bis 12 Gew.-% Ibuprofen unter Rühren zu dem Stoff gemäß (a),
nachfolgend Erwärmen des Gemisches unter Rühren auf 60 bis 75 °C;
(c) Lösen von 0,05 bis 2 Gew.-% Methyl-4-hydroxybenzoät, Natrium und 1 bis 5 Gew.-% 1,2-Propandiol in 10 bis 50 Gew.-% gereinigtem Wasser bei 60 bis 75 °C;
(d) Zugeben der wässrigen Lösung gemäß (c) zu der Lösung gemäß (b) unter Rühren zwecks Erhalt einer Emulsion;
(e) Suspendieren von 0,1 bis 1 Gew.-% Xanthan-Gummi, 1 bis 5 Gew.-% 1,2-Propandiol und 0,1 bis 2,5 Gew.-% Benzylnicotinat oder Capsaicin;
(f) Zugeben der Suspension gemäß (e) zu der Emulsion gemäß (d) unter Rühren;
(g) Homogenisieren der Emulsion gemäß (f), Abkühlen der Emulsion unter Rühren.

Wie zuvor erwähnt werden gemäß einer bevorzugten Ausführungsform der Erfindung im Schritt (c) folgende Unterschritte durchgeführt:
(c1) Lösen von 0,05 bis 2 Gew.-% Methyl-4-hydroxybenzoat, Natrium in 1 bis 5 Gew.-% Wasser;
(c2) Erwärmen von 9 bis 45 Gewichtsprozent Wasser auf 60 bis 75 °C;
(c3) Zugeben von 1 bis 5 Gew.-% 1,2-Propandiol in das Wasser gemäß (c2);
(c4) Mischen der Lösungen gemäß (c1) und (c3).

Gemäß einer besonders bevorzugten verfahrenstechnischen Ausführungsform erfolgen im Schritt (d) ein erstes Homogenisieren über einen Zeitraum von 18 bis 23 Min., anschließend ein Abkühlen der Emulsion auf 50 °C, nachfolgend ein zweites Homogenisieren über einen Zeitraum von 7,5 Min. und anschließend ein Abkühlen der Emulsion auf 42 °C.

Gemäß einer weiteren, besonders bevorzugten verfahrenstechnischen Ausführungsform erfolgen im Schritt (g) ein erstes Homogenisieren über einen Zeitraum von 1 Min. und ein zweites Homogenisieren über einen Zeitraum von 5 Min. und ein Abkühlen auf 34 °C.

Wie zuvor bereits angedeutet wird durch die Verwendung von letztlich drei Emulgatoren eine zusätzliche Homogenisierstufe genutzt, um eine Erhöhung der Stabilität der Creme herbeizuführen. Erfindungsgemäß erfolgt die zusätzliche Homogenisation anders als üblich nicht bei 70 °C sondern lediglich bei, wie oben erwähnt, 50 °C. Dadurch lässt sich auch eine Verringerung des Energieverbrauchs erreichen.

In der beigefügten Figur ist schematisch ein Fließbild einer bevorzugten Ausführungsform der Erfindung in Bezug auf den Wirkstoff Benzylnicotinat dargestellt. Wie zuvor erwähnt kann anstelle des Wirkstoffs Benzylnicotinat auch der Wirkstoff Capsaicin zum Einsatz kommen. In dem Fließbild wird davon ausgegangen, dass das erfindungsgemäße Verfahren apparatetechnisch unter Vakuum, vorzugsweise unter einem Vakuum von 0,4 bis 0,7 bar, durchgeführt wird.

Zunächst werden gemäß Schritt (a) 30 bis 50 Gew.-%, besonders bevorzugt 41,69 Gew.-% mittelkettige Triglyceride, zum Beispiel Miglyol 812, bereitgestellt und in einen nicht näher dargestellten Vakuummischer und Homogenisierapparat eingesaugt.
4 bis 10 Gew.-%, besonders bevorzugt 7 Gew.-% Glycerolmonostearat, 1 bis 5 Gew.-%, besonders bevorzugt 2,4 Gew.-% Poly(oxyethylen)-30-stearat, 2 bis 8 Gew.-%, besonders bevorzugt 4,2 Gew.-% Poly(oxyethylen)-100-stearat und 2 bis 12 Gew.-%, besonders bevorzugt 5 Gew.-% Ibuprofen werden im Schritt (b) unter Rühren in den Apparat gemäß Schritt (a) eingesaugt. Anschließend erfolgt ein Erwärmen des Gemisches unter Rühren auf 60 bis 75 °C. Das Rühren erfolgt generell beispielsweise bei 20 Umdrehungen/Min.

Im Schritt (c) wird eine Gesamtlösung bestehend aus 0,05 bis 2 Gew.-%, besonders bevorzugt 1,5 Gew.-% Methyl-4-hydroxybenzoat-Natrium in 1 bis 5 Gew.-%, bevorzugt 3 Gew.-% Wasser, welches auf 60 bis 75 °C erhitzt wurde, gelöst und in eine Mischung bestehend aus 9 bis 45 Gew.-%, bevorzugt 32,7 Gew.-% Wasser, welches auf 60 bis 75 °C erwärmt wurde, und 1 bis 5 Gew.-%, besonders bevorzugt 2,5 Gew.-% 1,2-Propandiol hinzugefügt. Die im Schritt (c) hergestellte Gesamtlösung kann als Konservierungsmittellösung bezeichnet werden.

Im Schritt (d) wird die Lösung gemäß (c) in den Apparat gemäß (b) zwecks Bildung einer Emulsion eingesaugt. Nachfolgend erfolgt unter Rühren ein erstes Homogenisieren über einen Zeitraum von 18 bis 23 Min., anschließend ein Abkühlen auf 50 °C, sodann ein zweites Homogenisieren über einen Zeitraum von 7,5 Min. (450 sec) und anschließend ein Abkühlen der Emulsion auf 42 °C.

Im Schritt (e) werden 0,1 bis 1 Gew.-%, besonders bevorzugt 0,54 Gew.-% Xanthan-Gummi in einer Mischung aus 1 bis 5 Gew.-%, besonders bevorzugt 2,5 Gew.-% 1,2-Propandiol und 0,1 bis 2,5 Gew.-%, besonders bevorzugt 0,5 oder 1 Gew.-% Benzylnicotinat suspendiert.

Im Schritt (f) wird die Suspension gemäß (e) in den Apparat, in dem die Emulsion gemäß (d) vorliegt, unter Rühren eingesaugt.

Im Schritt (g) wird unter Rühren die Phase gemäß (f) zunächst in einer ersten Homogenisierungsstufe über einen Zeitraum von 1 Min. (60 sec) homogenisiert, anschließend erfolgt ein zweites Homogenisieren (zweite Homogenisierungsstufe) über einen Zeitraum von 5 Min. (300 sec) und ein Abkühlen der Phase auf 34 °C.

Es wird eine glatte, weiß-cremefarbene, weiche Creme mit charakteristischem Geruch hergestellt. Anschließend wird die Creme mit Druckluft unter Rühren beispielsweise in einen Polyethylen-Inliner gefüllt.

Damit ist eine äußerlich anwendbare Zusammensetzung geschaffen, die insbesondere als wirksame Creme besonders stabil und haltbar vorliegen kann. Ferner ist ein Verfahren zum Herstellen einer vorgenannten Zusammensetzung angegeben.

## Patentansprüche

1. Äußerlich anwendbare Zusammensetzung mit einer wirksamen Menge Ibuprofen sowie folgenden weiteren Inhaltsstoffen:
mittelkettige Triglyceride,
Glycerolmonostearat,
Poly(oxyethylen)-30-stearat,
Poly(oxyethylen)-1 00-stearat,
Wasser,
Methyl-4-hydroxybenzoat, Natrium,
1,2-Propandiol,
Xanthan-Gummi,
wobei die Zusammensetzung ferner Benzylnicotinat oder Capsaicin enthält.

2. Zusammensetzung nach Anspruch 1, **dadurch gekennzeichnet, dass** die Inhaltsstoffe wie folgt vorliegen:
2 bis 12 Gew.-%, vorzugsweise 3 bis 8 Gew.-%, besonders bevorzugt 5,0 Gew.-% Ibuprofen,
30 bis 50 Gew.-%, vorzugsweise 35 bis 45 Gew.-%, besonders bevorzugt 41,69 Gew.-% mittelkettige Triglyceride,
4 bis 10 Gew.-%, vorzugsweise 5 bis 8 Gew.-%, besonders bevorzugt 7 Gew.-% Glycerolmonostearat,
1 bis 5 Gew.-%, vorzugsweise 2 bis 3 Gew.-%, besonders bevorzugt 2,4 Gew.-% Poly(oxyethylen)-30-stearat,
2 bis 8 Gew.-%, vorzugsweise 3 bis 5 Gew.-%, besonders bevorzugt 4,2 Gew.-% Poly(oxyethylen)-1 00-stearat,
10 bis 50 Gew.-%, vorzugsweise 20 bis 40 Gew.-%, besonders bevorzugt 32,85 bis 33,35 Wasser (gereinigt),
0,05 bis 0,4 Gew.-%, vorzugsweise 0,1 bis 0,2 Gew.-%, besonders bevorzugt 0,15 Gew.-% Methyl-4-hydroxybenzoat, Natrium,
2 bis 10 Gew.-%, vorzugsweise 3 bis 6 Gew.-%, besonders bevorzugt 5,0 Gew.-% 1,2-Propandiol,
0,1 bis 1 Gew.-%, vorzugsweise 0,2 bis 0,8 Gew.-%, besonders bevorzugt 0,54 Gew.-% Xanthan-Gummi,
0,1 bis 2,5 Gew.-%, vorzugsweise 0,3 bis 1,5 Gew.-%, besonders bevorzugt 0,5 bis 1,0 Gew.-% Benzylnicotinat oder Capsaicin.

3. Zusammensetzung nach Anspruch 1 oder 2, **gekennzeichnet durch** den weiteren Inhaltsstoff Parfüm, vorzugsweise Lavendelöl und Bitterorangenblütenöl.

4. Zusammensetzung nach Anspruch 3, **dadurch gekennzeichnet, dass** die Zusammensetzung 0,05 bis 0,18 Gew.-%, bevorzugt 0,08 bis 0,15 Gew.-%, besonders bevorzugt 0,11 Gew.-% Lavendelöl und 0,01 bis 0,12 Gew.-%, bevorzugt 0,04 bis 0,08 Gew.-%, besonders bevorzugt 0,06 Gew.-% Bitterorangenblütenöl enthält.

5. Zusammensetzung nach einem der vorhergehenden Ansprüche, **dadurch gekennzeichnet, dass** sie in Form einer Creme vorliegt.

6. Verfahren zum Herstellen einer äußerlich anwendbaren Zusammensetzung nach einem der vorhergehenden Ansprüche, **gekennzeichnet durch** die folgenden Schritte:
(a) Bereitstellen von 30 bis 50 Gew.-% mittelkettige Triglyceride;
(b) Zugeben von 4 bis 10 Gew.-% Glycerolmonostearat, 1 bis 5 Gew.-% Poly(oxyethylen)-30-stearat, 2 bis 8 Gew.-% Poly(oxyethylen)-100-stearat und 2 bis 12 Gew.-% Ibuprofen unter Rühren zu dem Stoff gemäß (a),
nachfolgend Erwärmen des Gemisches unter Rühren auf 60 bis 75 °C;
(c) Lösen von 0,05 bis 2 Gew.-% Methyl-4-hydroxybenzoat, Natrium und 1 bis 5 Gew.-% 1,2-Propandiol in 10 bis 50 Gew.-% gereinigtem Wasser bei 60 bis 75 °C;
(d) Zugeben der wässrigen Lösung gemäß (c) zu der Lösung gemäß (b) unter Rühren zwecks Erhalt einer Emulsion;
(e) Suspendieren von 0,1 bis 1 Gew.-% Xanthan-Gummi, 1 bis 5 Gew.-% 1,2-Propandiol und 0,1 bis 2,5 Gew.-% Benzylnicotinat oder Capsaicin;
(f) Zugeben der Suspension gemäß (e) zu der Emulsion gemäß (d) unter Rühren;
(g) Homogenisieren der Emulsion gemäß (f), Abkühlen der Emulsion unter Rühren.

7. Verfahren nach Anspruch 6, **dadurch gekennzeichnet, dass** im Schritt (c) folgende Unterschritte durchgeführt werden:
(c1) Lösen von 0,05 bis 2 Gew.-% Methyl-4-hydroxybenzoat, Natrium in 1 bis 5 Gew.-% Wasser;
(c2) Erwärmen von 9 bis 45 Gewichtsprozent Wasser auf 60 bis 75 °C;
(c3) Zugeben von 1 bis 5 Gew.-% 1,2-Propandiol in das Wasser gemäß (c2);
(c4) Mischen der Lösungen gemäß (c1) und (c3).

8. Verfahren nach Anspruch 6 oder 7, **dadurch gekennzeichnet, dass** im Schritt (d) ein erstes Homogenisieren über einen Zeitraum von 18 bis 23 Min., anschließend ein Abkühlen der Emulsion auf 50 °C,
nachfolgend ein zweites Homogenisieren über einen Zeitraum von 7,5 Min. und anschließend ein Abkühlen der Emulsion auf 42 °C erfolgen.

9. Verfahren nach einem der Ansprüche 6 bis 8, **dadurch gekennzeichnet, dass** im Schritt (g) ein erstes Homogenisieren über einen Zeitraum von 1 Min. und ein zweites Homogenisieren über einen Zeitraum von 5 Min. und das Abkühlen auf 34 °C erfolgen.

10. Verfahren nach einem der Ansprüche 6 bis 9, **dadurch gekennzeichnet, dass** es unter Vakuum, vorzugsweise unter einem Vakuum von 0,4 bis 0,7 bar, durchgeführt wird.
